# EUROPEAN PATENT SPECIFICATION

(11) **EP 3 603 669 B1**
(45) Date of publication and mention of the grant of the patent: **19.06.2024**
(21) Application number: 18775644.0
(22) Date of filing: 30.03.2018
(51) Int. Cl.: A61K 39/395, A61K 9/08, A61K 47/18, A61K 47/26, A61P 17/06, A61P 37/02

(54) **AQUEOUS FORMULATION, AQUEOUS FORMULATION IN INJECTOR, ANTIBODY PROTEIN DISAGGREGATING AGENT, AND ANTIBODY PROTEIN DISAGGREGATION METHOD**
WÄSSRIGE FORMULIERUNG, WÄSSRIGE FORMULIERUNG IN EINEM INJEKTOR, ANTIKÖRPERPROTEINDISAGGREGATIONSMITTEL UND ANTIKÖRPERPROTEINDISAGGREGATIONSVERFAHREN
FORMULATION AQUEUSE, FORMULATION AQUEUSE DANS UN INJECTEUR, AGENT DE DÉSAGRÉGATION DE PROTÉINE D'ANTICORPS ET PROCÉDÉ DE DÉSAGRÉGATION DE PROTÉINE D'ANTICORPS

(30) Priority: 31.03.2017 JP 2017070844
(43) Date of publication of application: 05.02.2020
(73) Proprietor: Meiji Seika Pharma Co., Ltd., Tokyo 104-8002 (JP); Dong-A Socio Holdings Co., Ltd., Seoul 02587 (KR)
(72) Inventor: KAYA, Arpansiree, Odawara-shi Kanagawa 250-0852 (JP); FUJITA, Michinari, Odawara-shi Kanagawa 250-0852 (JP)
(74) Representative: J A Kemp LLP
(86) International application number: PCT/JP2018/013559
(87) International publication number: WO 2018/181876

(56) References cited:
- WO-A2-2007/076062
- WO-A2-2009/114040
- WO-A2-2009/114040
- JP-A- 2005 508 981
- JP-A- 2012 510 468
- JP-A- 2013 544 763
- JP-A- 2016 505 572
- Janssen: "STELARA 45 mg Injektionslösung in einer Fertigspritze", , 1 July 2011 (2011-07-01), pages 1-5, XP055467870, Retrieved from the Internet: URL:https://www.praxis-kiedrowski.de/files /fachinformation_stelara.pdf [retrieved on 2018-04-17]
- JACQUELINE M. BENSON ET AL: "Discovery and mechanism of ustekinumab: A human monoclonal antibody targeting interleukin-12 and interleukin-23 for treatment of immune-mediated disorders", MABS, vol. 3, no. 6, 1 November 2011 (2011-11-01), pages 535-545, XP055133436, ISSN: 1942-0862, DOI: 10.4161/mabs.3.6.17815
- CHEN BEI ET AL: "Influence of histidine on the stability and physical properties of a fully human antibody in aqueous and solid forms", PHARMACEUTICAL RESEARCH, SPRINGER NEW YORK LLC, US, vol. 20, no. 12, 1 December 2003 (2003-12-01), pages 1952-1960, XP002386671, ISSN: 0724-8741, DOI: 10.1023/B:PHAM.0000008042.15988.C0
- DING C ET AL: "ABT-874, a fully human monoclonal anti-IL-12/IL-23 antibody for the potential treatment of autoimmune diseases", CURRENT OPINION IN INVESTIGATIONAL DRUGS, CURRENT DRUGS, LONDON, GB, vol. 9, no. 5, 1 January 2008 (2008-01-01) , pages 515-522, XP008134761, ISSN: 0967-8298
- KALONIA, C. et al.: "Effects of Protein Conformation, Apparent Solubility, and Protein-Protein Interactions on the Rates and Mechanisms of Aggregation for an IgG1 Monoclonal Antibody", J. Physical Chemistry B, vol. 120, no. 29, 5 July 2016 (2016-07-05) , pages 7062-7075, XP055612741,

## Description

### [Technical Field]

The present invention relates to an aqueous formulation and an in-syringe aqueous formulation as well as an antibody protein deaggregation method using the same. In particular, the present invention relates to an ustekinumab-containing aqueous formulation and an in-syringe aqueous formulation containing the same, as well as an antibody protein deaggregation method.

### [Background Art]

Ustekinumab is a generic name for a human type anti-human IL-12/23 p40 monoclonal antibody, that is, a human IgG1 monoclonal antibody to a p40 subunit which is a constituent protein common to human IL-12 (interleukin-12) and IL-23 (interleukin-23). It is known that the human IL-12 is involved in the differentiation of CD4 positive naive T cells into helper T cells 1 (Th1), and the IL-23 promotes the activation of helper T cells 17 (Th17). Ustekinumab, which has high affinity and selectivity for such p40 subunit of human IL-12 and IL-23 (IL-12/23 p40), is used as a polypeptide (antibody protein) drug for the treatment of autoimmune diseases such as psoriasis vulgaris and arthritic psoriasis, and is generally administered by injection. Ustekinumab is produced by genetic recombination technology in mammalian cell expression systems such as Chinese hamster ovary cells and mouse myeloma (Sp2/0) cells described in, for example, International Publication No. WO2009/114040 (PTL 1). In addition, an aqueous formulation (injection) containing ustekinumab is produced and marketed by Janssen Pharmaceutical K.K. as "Stelara (registered trademark)," for example.

Meanwhile, an aqueous formulation containing an antibody protein such as ustekinumab is susceptible to chemical changes and physical changes such as aggregation during production process and storage due to the complex structure of the antibody protein. Such instability of the formulation not only reduces the pharmacological action of the antibody protein but also greatly affects the safety for the patient, and therefore, particularly excellent stability is required for antibody protein-containing aqueous formulations.

For the purpose of stabilizing the antibody protein-containing aqueous formulation, studies have been made on, for example, the addition of additives such as saccharides, amino acids, water-soluble polymers, surfactants, and buffer agents, and on various conditions such as dosage form, pH, and concentrations of components. For example, International Application Japanese-Phase Publication No. 2016-65079 (PTL 2) describes an antibody-containing solution formulation containing an antibody, 40 to 1000 mM arginine, and 10 to 200 mM methionine in a buffer solution, and Japanese Unexamined Patent Application Publication No. 2016-117756 (PTL 3) describes an antibody-containing formulation containing basic amino acid-aspartate or basic amino acid-glutamate. In addition, Japanese Unexamined Patent Application Publication No. 2016-65091 (PTL 4) describes a pharmaceutical formulation containing a monoclonal antibody in a histidine-acetate buffer solution (pH 5.5 to 6.5), and International Application Japanese-Phase Publication No. 2007-524602 (PTL 5) describes a liquid formulation containing 100 to 260 mg/ml protein or antibody, 50 to 200 mM arginine-HCl, 10 to 100 mM histidine, and 0.01 to 0.1% polysorbate. However, these PTLs 2 to 5 do not describe anything about ustekinumab.

In addition, with regard to antibody proteins, studies have been made also on methods of solubilizing and regenerating aggregated antibody proteins. For example, International Application Japanese-Phase Publication No. Hei 11-514334 (PTL 6) describes a method for regenerating TFPI by adding a charged polymer solution to a denatured TFPI (tissue factor pathway inhibitor), and Japanese Unexamined Patent Application Publication No. Hei 10-522270 (PTL 7) describes a method for refolding a denatured protein by selecting a pH and an antibody binding constant. However, these PTLs 6 and 7 do not describe anything about ustekinumab, either. Moreover, the methods described in these literatures are all methods for solubilizing and/or regenerating aggregates formed by improper folding in the process of genetic engineering production of an antibody protein, and PTLs 6 and 7 do not describe any method for deaggregating an antibody protein aggregated in the aqueous formulation due to stress such as heat during production process or storage.

The package insert of STELARA^{®} 45 mg (Janssen^{®}) (URL:https://www.praxiskiedrowski.de/files/fachinforma tion_stelara.pdf) (1 July 2011 (2011-07-01), pages 1-5) (Non-PTL 1) discloses a STELARA 45 mg solution for injection in a pre-filled syringe.

Jacqueline M. Benson et al. (mAbs, vol. 3, no. 6, 1 November 2011 (2011 -11 -01), pages 535-545) (Non-PTL 2) reports discovery and mechanism of ustekinumab as a human monoclonal antibody targeting interleukin-12 and interleukin-23 for treatment of immune-mediated disorders.

WO 2009/114040 A2 (PTL 8) discloses ANTI-IL-12/23P40 antibodies, epitopes, formulations, compositions, methods and uses thereof.

CHEN BEI et al. (PHARMACEUTICAL RESEARCH, SPRINGER NEW YORK LLC, US, vol. 20, no. 12, 1 December 2003 (2003-12-01), pages 1952-1960) (Non-PTL 3) reports influence of histidine on the stability and physical properties of a fully human antibody in aqueous and solid forms.

WO 2007/076062 A2 (PTL 9) discloses protein formulations with reduced viscosity and uses thereof.

DING C et al. (CURRENT OPINION IN INVESTIGATIONAL DRUGS, CURRENT DRUGS, LONDON, GB, vol. 9, no. 5, 1 January 2008 (2008-01-01), pages 515-522) (Non-PTL 4) reports ABT-874 as a fully human monoclonal anti-IL-12/IL-23 antibody for the potential treatment of autoimmune diseases.

### [Citation List]

### [Patent Literature]

[PTL 1] International Publication No. WO2009/114040
[PTL 2] International Application Japanese-Phase Publication No. 2016-65079
[PTL 3] Japanese Unexamined Patent Application Publication No. 2016-117756
[PTL 4] Japanese Unexamined Patent Application Publication No. 2016-65091
[PTL 5] International Application Japanese-Phase Publication No. 2007-524602
[PTL 6] International Application Japanese-Phase Publication No. Hei 11-514334
[PTL 7] Japanese Unexamined Patent Application Publication No. Hei 10-522270
[PTL 8] International Publication No. WO 2009/114040
[PTL 9] International Publication No. WO 2007/076062

### [Non-Patent Literature]

[Non-PTL 1] Package insert of STELARA® 45 mg (https: //www.praxiskiedrowski.de/files/fachinformation_stelar a.pdf)
[Non-PTL 2] Jacqueline M. Benson et al., mAbs, vol. 3, no. 6, 1 November 2011 (2011 -11 -01), pages 535-545
[Non-PTL 3] CHEN BEI et al., PHARMACEUTICAL RESEARCH, SPRINGER NEW YORK LLC, US, vol. 20, no. 12, 1 December 2003 (2003-12-01), pages 1952-1960
[Non-PTL 4] DING C et al., CURRENT OPINION IN INVESTIGATIONAL DRUGS, CURRENT DRUGS, LONDON, GB, vol. 9, no. 5, 1 January 2008 (2008-01-01), pages 515-522

### [Technical Problem]

The present invention has been made in light of the above problems, and an object thereof is to provide an ustekinumab-containing aqueous formulation excellent in stability and an in-syringe aqueous formulation containing the same, and an antibody protein deaggregation method.

### [Solution to Problem]

The present inventors have made earnest studies to achieve the above object, and have found as a result that the generation of ustekinumab aggregates (aggregated ustekinumab) is sufficiently suppressed when ustekinumab is formulated with a high-concentration histidine buffer solution to form an ustekinumab-containing aqueous formulation containing ustekinumab, at least one selected from histidine and salts thereof, and water in combination. Surprisingly, the present inventors have further found that, when the high-concentration histidine buffer solution is blended in aggregated ustekinumab which has been aggregated due to stress such as heat after genetic engineering production, the aggregated ustekinumab is deaggregated, making it possible to obtain the ustekinumab-containing aqueous formulation in which the generation of aggregated ustekinumab is sufficiently suppressed.

### [Summary of the Invention]

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

Aspects of the present invention obtained by the above knowledge are as follows.
(1) An aqueous formulation comprising:
   ustekinumab; at least one selected from histidine and salts thereof; and water, wherein a content of the ustekinumab is 80 to 100 mg/mL, a content of the histidine and/or the salts thereof is 50 to 90 mM in terms of histidine, and a pH thereof is 5.7 to 6.3.
(2) The aqueous formulation according to (1), further comprising methionine, wherein a content of the methionine is 10 to 50 mM.
(3) The aqueous formulation according to (1) or (2), further comprising sucrose.
(4) The aqueous formulation according to any one of (1) to (3), formed by blending the ustekinumab and a histidine buffer solution.
(5) An in-syringe aqueous formulation comprising a syringe and the aqueous formulation according to any one of (1) to (4) filled in the syringe.
(6) An antibody protein deaggregation method for deaggregating aggregated ustekinumab, comprising the step of: adding an antibody protein deaggregation agent to the aggregated ustekinumab to obtain an aqueous formulation according to the invention, wherein the antibody protein deaggregation agent deaggregates aggregated ustekinumab and comprises: at least one selected from histidine and salts thereof; and water, wherein a content of the histidine and/or the salts thereof is 50 to 90 mM in terms of histidine, and a pH thereof is 5.7 to 6.3.
(7) The antibody protein deaggregation method of (6), wherein the antibody protein deaggregation agent further comprises methionine, wherein a content of the methionine is 5 to 50 mM.
(8) The antibody protein deaggregation method of (6) or (7), wherein the antibody protein deaggregation agent further comprises sucrose.

### [Advantageous Effects of Invention]

The present invention makes it possible to provide an ustekinumab-containing aqueous formulation excellent in stability and an in-syringe aqueous formulation containing the same, and an antibody protein deaggregation method.

### [Brief Description of Drawings]

[Fig. 1] Fig. 1 is a graph illustrating relative aggregate amounts after storing aqueous formulations obtained in Example 1 and Comparative Examples 1 to 9 at 40°C for 2 weeks.
[Fig. 2] Fig. 2 is a graph illustrating relative aggregate amounts after storing aqueous formulations obtained in Comparative Examples 10 and 11 at 40°C for 2 weeks.
[Fig. 3] Fig. 3 is a graph illustrating %aggregate after each of the aqueous formulations obtained in Examples 1 to 3 and Comparative Examples 2 and 12 to 14 was stored at 40°C for 2 weeks.
[Fig. 4] Fig. 4 is a graph illustrating the relationship between the %aggregate and the methionine concentration after storage at 40°C for 2 weeks in the aqueous formulations obtained in Examples 1 to 3.
[Fig. 5] Fig. 5 is a graph illustrating the relationship between the %aggregate and the storage time at 40°C in the aqueous formulations obtained in Examples 4 and 5 and Comparative Example 1.
[Fig. 6] Fig. 6 is a graph illustrating the relationship between the %aggregate and the storage time at 40°C in the aqueous formulations obtained in Examples 6 to 9 and Comparative Example 15.
[Fig. 7] Fig. 7 is a graph illustrating the relationship between the %monomer and the storage time at 40°C in the aqueous formulations obtained in Examples 6 to 9 and Comparative Example 15.

### [Detailed Description]

An aqueous formulation of the present invention comprises ustekinumab, at least one selected from histidine and salts thereof, and water, wherein a content of the ustekinumab is 80 to 100 mg/mL, a content of the histidine and/or the salts thereof is 50 to 90 mM in terms of histidine, and a pH thereof is 5.7 to 6.3.

In addition, an antibody protein deaggregation agent of the disclosure is an antibody protein deaggregation agent which deaggregates aggregated ustekinumab, comprising at least one selected from histidine and salts thereof, and water, wherein a content of the histidine and/or the salts thereof is 50 to 90 mM in terms of histidine.

Moreover, an antibody protein deaggregation method of the present invention comprises the step of adding the antibody protein deaggregation agent to the aggregated ustekinumab.

The "aqueous formulation" in the present invention refers to a formulation containing water as a solvent, among formulations (pharmaceutical formulations) prepared to be suitable for administration to a patient in need of treatment. The water is not particularly limited as long as it is pharmaceutically acceptable water. The aqueous formulation of the present invention is preferably an injection or an infusion fluid. In addition, the "ustekinumab-containing aqueous formulation" in the disclosure refers to a formulation containing at least water and ustekinumab, among the aqueous formulations.

The "antibody" in the present invention is not particularly limited as long as it binds to a desired antigen, and may be a polyclonal antibody or a monoclonal antibody, but is preferably a monoclonal antibody from the viewpoint of homogeneity and stability. In addition, the "antibody protein" in the present invention refers to a protein which functions as an antibody, and also includes, in addition to the antibodies in general, antibody fragments and depolymerized antibodies bound with antibody variable regions.

In the present invention, the antibody protein includes ustekinumab. The "ustekinumab" in the present invention is a generic name for a human IgG1 monoclonal antibody (human type anti-human IL-12/23 p40 monoclonal antibody) to a p40 subunit which is a common constituent protein of human IL-12 (interleukin-12) and IL-23 (interleukin-23), and the monomer being its active form is composed of 2 molecules of H-chains (γ1 chain) each composed of 449 amino acid residues and 2 molecules of L-chains (κ chain) each composed of 214 amino acid residues, and has a molecular weight of about 148 to 149.7 kDa. Ustekinumab is conventionally known, and is also known as an active ingredient of "Stelara (registered trademark)" commercially available as an injection. The "ustekinumab" in the present invention includes proteins pharmaceutically equivalent to the human type anti-human IL-12/23 p40 monoclonal antibody being the active ingredient of "Stelara (registered trademark)," and the active ingredients of the biosimilars of "Stelara (registered trademark)" and pharmaceutically equivalent proteins thereof.

Ustekinumab can be produced by appropriately employing and improving conventionally known methods, and can be produced by genetic recombination technology in mammalian cell expression systems such as Chinese hamster ovary cells in accordance with the method described in, for example, WO 2009/114040 (PTL 1) .

In addition, the "ustekinumab" according to the present invention includes both unaggregated ustekinumab and aggregated ustekinumab unless otherwise specified. In the disclosure, the "unaggregated ustekinumab" refers to an antibody molecule composed of two heavy chain molecules and two light chain molecules, and is described as a monomer. In addition, the "aggregated ustekinumab" in the present invention refers to an ustekinumab aggregate, and examples thereof include ustekinumab which is produced by the genetic recombination technology (unaggregated ustekinumab) and is then denatured and aggregated due to the exposure to an inappropriate environment such as high temperature, low temperature, pressure, acid, or base, or for example an environment with a temperature of 25 to 40°C followed by formation into a dimer or a trimer.

Moreover, the "antibody protein deaggregation agent" and the "antibody protein deaggregation method" in the present invention refers to an agent which has a function of deaggregating an aggregated antibody protein and which is used for that purpose, and a method capable of deaggregating an aggregated antibody protein, respectively. The "deaggregation" refers to a phenomenon in which an antibody protein, which has been denatured and aggregated due to the exposure to the above inappropriate environment and formed into a dimer or a trimer (in the present invention, the aggregated ustekinumab), is dissociated to return to an unaggregated state (in the disclosure, the unaggregated ustekinumab). Note that, unaggregated ustekinumab and aggregated ustekinumab can be distinguished by size exclusion chromatography (SEC). In the aqueous formulation, the above-described ustekinumab is preferably unaggregated ustekinumab from the viewpoint of safety. However, since the aqueous formulation of the present invention can suppress the aggregation of unaggregated ustekinumab and deaggregate aggregated ustekinumab, it is possible to sufficiently reduce the content of the aggregated ustekinumab in the entire ustekinumab (preferably less than 1.0% by mass).

In the aqueous formulation of the present invention, the content of the ustekinumab (the content of the unaggregated ustekinumab or the content of the aggregated ustekinumab, or the total content of them if both of them are contained, hereinafter the same) is 80 to 100 mg/mL. In the present invention, the generation of aggregates is sufficiently suppressed even when the concentration of ustekinumab in the aqueous formulation is high as above. The content of the ustekinumab may be 80 mg/mL, 90 mg/mL, or 100g/mL, and particularly preferably 90 mg/mL.

In addition, in the antibody protein deaggregation agent of the disclosure and the antibody protein deaggregation method of the present invention, it is more preferable that the antibody protein deaggregation agent be added to the aggregated ustekinumab such that the content of the ustekinumab in the obtained aqueous formulation is in the above range.

The aqueous formulation of the present invention and the antibody protein deaggregation agent of the disclosure contain at least one selected from histidine and salts thereof. Examples of histidine include the D-form, the L-form, and the DL-form, and the L-form is preferable among these. In addition, the salt of histidine (histidine salt) is preferably a pharmaceutically acceptable salt, and examples thereof include histidine hydrochloride and hydrates thereof.

In the aqueous formulation of the present invention and the antibody protein deaggregation agent of the disclosure, the content of the histidine and/or the salt thereof (the content of the histidine or the content of the histidine salt, or the total content of them if both histidine and the salt thereof are contained, hereinafter the same) needs to be 50 to 90 mM in terms of histidine. The present inventors have found that the combination of histidine and/or a salt thereof at a high concentration with ustekinumab particularly suppresses the aggregation of the ustekinumab and further deaggregates the ustekinumab when the ustekinumab is aggregated ustekinumab.

The content of the histidine and/or the salt thereof is more preferably 60 to 80 mM and further preferably 60 to 70 mM. In addition, the content of the histidine and/or the salt thereof is more preferably 60 to 80 mM and further preferably 60 to 70 mM also in the case where the aqueous formulation of the present invention and the antibody protein deaggregation agent of the disclosure further contain the methionine to be described later. When the content of the histidine and/or the salt thereof is less than the lower limit, the effect of suppressing the aggregation of unaggregated ustekinumab (hereinafter simply referred to as the "aggregation suppression effect" in some cases) and the effect of deaggregating aggregated ustekinumab (hereinafter simply referred to as the "deaggregation effect" in some cases) tend to decrease. On the other hand, when the upper limit is exceeded, the suitability as a pharmaceutical formulation tends to decrease due to coloring caused by the oxidation of histidine, an increasing in osmotic pressure, and the like.

In addition, in the antibody protein deaggregation agent of the disclosure and the antibody protein deaggregation method of the present invention, the antibody protein deaggregation agent is added to the aggregated ustekinumab such that the content of the histidine and/or the salt thereof in the obtained aqueous formulation is in the above range.

Moreover, the aqueous formulation of the present invention and the antibody protein deaggregation agent of the disclosure preferably further contain methionine from the viewpoint of further improving the aggregation suppression effect and the deaggregation effect. Examples of methionine include the D-form, the L-form, and the DL-form, and the L-form is preferable among these.

In the aqueous formulation of the present invention and the antibody protein deaggregation agent of the disclosure, the content of methionine is preferably 5 to 50 mM, more preferably 20 to 30 mM, and further preferably 25 mM. When the content of methionine is less than the lower limit, there is a tendency that further improvement of the aggregation suppression effect and the deaggregation effect cannot be expected. On the other hand, when the upper limit is exceeded, the suitability as a pharmaceutical formulation tends to decrease due to an increase in osmotic pressure and the like.

In addition, in the antibody protein deaggregation agent of the disclosure and the antibody protein deaggregation method of the present invention, it is more preferable that the antibody protein deaggregation agent be added to the aggregated ustekinumab such that the content of methionine in the obtained aqueous formulation is in the above range.

The pH of the aqueous formulation of the present invention and the antibody protein deaggregation agent of the disclosure is 5.7 to 6.3, and preferably 6.0. A pH in the above range tends to provide a better aggregation suppression effect and deaggregation effect. On the other hand, pain at the time of injection tends to easily occur when the pH is less than the above lower limit, and the aggregation suppression effect and the deaggregation effect tend to decrease when the upper limit is exceeded.

In addition, in the antibody protein deaggregation agent of the disclosure and the antibody protein deaggregation method of the present invention, it is more preferable that the antibody protein deaggregation agent be added to the aggregated ustekinumab such that the pH of the obtained aqueous formulation is in the above range.

The aqueous formulation of the present invention and the antibody protein deaggregation agent of the disclosure may be an agent formed by blending ustekinumab, at least one selected from histidine and salts thereof, water, and methionine as necessary, and may be an agent formed by blending ustekinumab, a histidine buffer solution, and methionine as necessary from the viewpoint that a better aggregation suppression effect and deaggregation effect tend to be provided. Specifically, it is preferable that the histidine and the salt thereof be derived from the histidine buffer solution.

The "buffer solution" in the present invention is a solution capable of maintaining the pH of the solution in an acceptable range by the action of its acid-base conjugate components. The "histidine buffer solution" in the present invention is a buffer solution formed by blending at least two selected from the group consisting of histidine and salts thereof (including two kinds of histidine salts), and water. The histidine and the salts thereof are as described above. The pH of the histidine buffer solution is preferably a pH at which the pH of each of the aqueous formulation of the present invention and the deaggregation agent of the disclosure is in the above-described preferable range.

The aqueous formulation of the present invention and the antibody protein deaggregation agent of the disclosure may further contain histidine and a salt thereof, and a compound having a pH buffer function other than methionine (hereinafter referred to as the "additional buffer agent").

Examples of the additional buffer agent include phosphoric acid, sodium phosphate, sodium dihydrogen phosphate, disodium hydrogen phosphate, potassium phosphate, potassium dihydrogen phosphate, dipotassium hydrogen phosphate, acetic acid, sodium acetate, potassium acetate, ammonium acetate, succinic acid, sodium succinate, citric acid, sodium citrate, potassium citrate, maleic acid, tartaric acid, sodium tartrate, lactic acid, sodium lactate, malic acid, boric acid, ascorbic acid, glutamic acid, sodium glutamate, arginine, arginine hydrochloride, tris-(hydroxymethyl)-aminomethane(tris), and diethanolamine, and these may be used singly or in combination of two or more kinds. In addition, these may be ones blended as a buffer solution (phosphate buffer solution, acetate buffer solution, succinate buffer solution, citrate buffer solution, arginine buffer solution, other organic acid buffer solutions, and the like).

In addition, the aqueous formulation of the present invention and the antibody protein deaggregation agent of the disclosure may further contain an additive such as a tonicity adjusting agent or an excipient as long as the effects thereof are not impaired. Among these, the aqueous formulation of the present invention and the antibody protein deaggregation agent of the disclosure preferably further contain the above-described tonicity adjusting agent.

The "tonicity adjusting agent" in the disclosure is a compound having a function of adjusting the osmotic pressure in a range acceptable as a formulation, and examples thereof include sodium chloride, potassium chloride, sorbitol, mannitol, sucrose, xylitol, trehalose, glucose, glycine, proline, and cysteine, and these may be used singly or in combination of two or more kinds. Among these, the tonicity adjusting agent is particularly preferably sucrose from the viewpoint that a better aggregation suppression effect and deaggregation effect tend to be provided.

Examples of the "excipient" in the disclosure include lactose, glycerol, maltose, inositol, bovine serum albumin (BSA), dextran, polyvinyl alcohol (PVA), hydroxypropyl methylcellulose (HPMC), polyethyleneimine, gelatin, polyvinyl pyrrolidone (PVP), hydroxyethyl cellulose (HEC), polyethylene glycol, ethylene glycol, dimethyl sulfoxide (DMSO), dimethylformamide (DMF), L-serine sodium glutamate, potassium glutamate, alanine, lysine hydrochloride, sarcosine, γ-aminobutyric acid, Polysorbate 20, Polysorbate 80, SDS, polyoxyethylene copolymers, sodium acetate, ammonium sulfate, magnesium sulfate, sodium sulfate, trimethylamine N-oxide, betaine, zinc ions, copper ions, calcium ions, manganese ions, magnesium ions, CHAPS, sucrose monolaurate, and 2-O-β-mannoglycerate, and these may be used singly or in combination of two or more kinds. Among these, the excipient preferably has a function as a surfactant and more preferably a polysorbate such as Polysorbate 20 or Polysorbate 80 from the viewpoint that ustekinumab can be protected against stress occurring at the air-liquid interface to further suppress its aggregation.

Note that, for example, arginine, sodium citrate, and the like among the additional buffer agents can also function as the above-described tonicity adjusting agent and that sorbitol, mannitol, sucrose, xylitol, trehalose, glucose, proline, and the like among the tonicity adjusting agents can also function as the above-described excipient.

Therefore, when the aqueous formulation of the present invention and the antibody protein deaggregation agent of the disclosure further contains the additional buffer agent, the tonicity adjusting agent, and the excipient, the content of each of them can be adjusted as appropriate as long as the effects thereof are not impaired, and the total content is such an amount that the osmotic pressure ratio of the aqueous formulation of the present invention (including the aqueous formulation obtained by adding the antibody protein deaggregation agent to the aggregated ustekinumab) to physiological saline is preferably 0.8 to 1.2 and particularly preferably 1.0.

For example, when the aqueous formulation of the present invention and the antibody protein deaggregation agent of the disclosure further contain sucrose, its content in the aqueous formulation and the antibody protein deaggregation agent is preferably 20 to 75 mg/mL, more preferably 40 to 55 mg/mL, and further preferably 44.7 to 51.8 mg/mL. The content of sucrose in the above range tends to provide a better aggregation suppression effect and deaggregation effect. On the other hand, the stability of the aqueous formulation tends to decrease when the content of sucrose is less than the lower limit, and the osmotic pressure of the aqueous formulation tends to be too high when the upper limit is exceeded.

In addition, for example, when the aqueous formulation of the present invention and the antibody protein deaggregation agent of the disclosure further contain a polysorbate, its content (total content in the case of a mixture) in the aqueous formulation and the antibody protein deaggregation agent is preferably 0.01 to 1 mg/mL, more preferably 0.01 to 0.1 mg/mL, and further preferably 0.04 mg/mL. The content of polysorbate in the above range tends to provide a better aggregation suppression effect and deaggregation effect. On the other hand, the stability of ustekinumab tends to decrease out of the above range.

Note that, in the antibody protein deaggregation agent of the disclosure and the antibody protein deaggregation method of the present invention, it is more preferable that the antibody protein deaggregation agent be added to the aggregated ustekinumab such that the contents of sucrose and polysorbate in the obtained aqueous formulation are in the above ranges.

The method for producing the aqueous formulation of the present invention and the antibody protein deaggregation agent of the disclosure is not particularly limited, and it is preferable to obtain the aqueous formulation of the present invention and the antibody protein deaggregation agent of the disclosure by blending ustekinumab, the histidine buffer solution, and methionine as necessary in the case of the aqueous formulation and by blending the histidine buffer solution and methionine as necessary in the case of the antibody protein deaggregation agent to the above-described concentration.

The aqueous formulation of the present invention is preferably used as an injection or an infusion fluid, and is preferably stored in a syringe such as a prefilled sterile syringe or in an infusion pack. The in-syringe aqueous formulation of the present invention preferably comprises a syringe and the aqueous formulation filled in the syringe.

In addition, the aqueous formulation of the present invention can also be a kit formulation with the specification manual of the aqueous formulation. The kit formulation of the disclosure preferably includes at least one of the aqueous formulation and the in-syringe aqueous formulation, and a specification manual of the aqueous formulation.

The antibody protein deaggregation method of the present invention includes adding the antibody protein deaggregation agent to aggregated ustekinumab such that the concentrations of the aggregated ustekinumab, the histidine and/or the salt thereof, and methionine as necessary are the above-described concentrations. In addition, the aggregated ustekinumab may be added with the histidine buffer solution and methionine as necessary such that their concentrations are the above-described concentrations. The antibody protein deaggregation agent of the disclosure and the antibody protein deaggregation method of the present invention make it possible to sufficiently deaggregate the aggregated ustekinumab. For this reason, the composition (aqueous formulation) obtained by adding the antibody protein deaggregation agent or the histidine buffer solution to the aggregated ustekinumab can be the aqueous formulation of the present invention as it is.

### [Examples]

### <Storage Stability Evaluation Test>

The aqueous formulations obtained in examples and comparative examples were subjected to size exclusion chromatography (SEC, column: TSKgel G3000SWXL 7.8 × 300 mm, manufactured by Tosoh Corporation, column temperature: 25°C, eluent: 20 mM Na₂HPO₄ + 150 mM NaCl (pH7.3), flow rate: 0.5 ml/min)) immediately after production and after storage for 2 weeks, 1 month, and 2 months at 40°C, to thereby measure the %aggregate (ratio of the aggregate mass to the total protein mass [%Aggregate]) and the %monomer (ratio of the monomer mass to the total protein mass [%Monomer]) in each aqueous formulation. Note that the smaller the value of the %aggregate or the larger the value of the %monomer, the aqueous formulation can be evaluated to be excellent in storage stability.

### (Example 1)

First, a histidine buffer solution (pH 6.0) was prepared from histidine and histidine hydrochloride monohydrate. Subsequently, ustekinumab and the histidine buffer solution were mixed to a composition of ustekinumab: 90 mg/mL and histidine and/or a salt thereof (in terms of histidine): 50 mM, to thereby obtain 500 µL of aqueous formulation. The ustekinumab sample used was obtained by purifying ustekinumab, expressed by using a Chinese hamster ovary cell stable expression strain in accordance with the method described in WO 2009/114040, by chromatography such as Protein A chromatography.

### (Comparative Example 1)

First, a histidine buffer solution (pH 6.0) was prepared in the same manner as in Example 1. Subsequently, an aqueous formulation was prepared to have the same composition as Stelara (registered trademark). Specifically, ustekinumab, the histidine buffer solution, Polysorbate 80, and sucrose were mixed to a composition of ustekinumab: 90 mg/mL, histidine and/or a salt thereof (in terms of histidine): 6.4 mM (1 mg/ml), Polysorbate 80: 0.04 mg/mL, and sucrose: 76 mg/mL, to thereby obtain 500 µL of aqueous formulation.

### (Comparative Example 2)

First, a histidine buffer solution (pH 6.0) was prepared in the same manner as in Example 1. Subsequently, ustekinumab and the histidine buffer solution were mixed to a composition of ustekinumab: 90 mg/mL and histidine and/or a salt thereof (in terms of histidine): 6.4 mM, to thereby obtain 500 µL of aqueous formulation.

### (Comparative Examples 3 to 9)

The aqueous formulations (ustekinumab: 90 mg/mL + histidine and/or a salt thereof (in terms of histidine): 6.4 mM + various amino acids) were obtained in the same manner as in Comparative Example 2 except that various amino acids were further mixed. The various amino acids used were such that Comparative Example 3: L-glutamate sodium salt (50 mM), Comparative Example 4: L-lysine hydrochloride (50 mM), Comparative Example 5: L-proline (50 mM), Comparative Example 6: L-leucine (30 mM (solubility limit concentration at the time of preparation of the formulation)), Comparative Example 7: L-tryptophan (10 mM (solubility limit concentration at the time of preparation of the formulation)), Comparative Example 8: glycine (50 mM), and Comparative Example 9: L-serine (50 mM) at the concentrations in the parentheses.

The storage stability evaluation test was carried out on each of the aqueous formulations obtained in Example 1 and Comparative Examples 1 to 9, and the following formula:
relative aggregate amount = (%aggregate after storing each aqueous formulation at 40°C for 2 weeks)/(%aggregate after storing the aqueous formulation of Comparative Example 2)

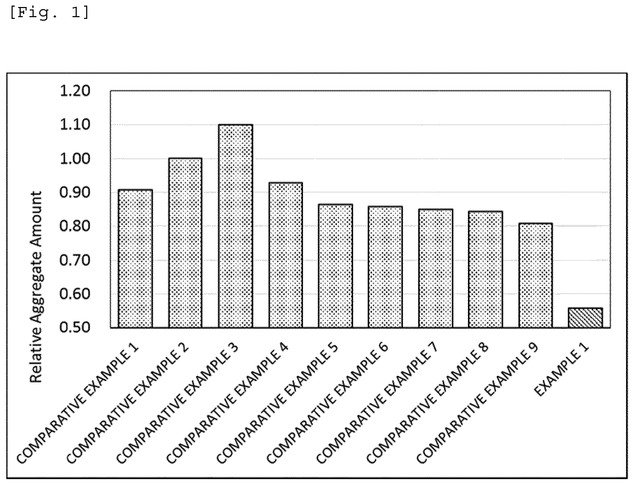

was used to obtain the relative aggregate amount after storing each aqueous formulation at 40°C for 2 weeks, that is, the relative amount of the %aggregate of each aqueous formulation to the %aggregate of the aqueous formulation obtained in Comparative Example 2 [Relative Aggregate Amount]. Fig. 1 illustrates a graph illustrating the relative aggregate amounts after storing the aqueous formulations at 40°C for 2 weeks. As is apparent from the results illustrated in Fig. 1, it was confirmed that the generation of aggregates (aggregated ustekinumab) was significantly suppressed in the aqueous formulation of the present invention (Example 1) formed by adding high-concentration histidine and/or a salt thereof to an ustekinumab-containing aqueous formulation.

### (Comparative Examples 10 and 11)

The aqueous formulations (ustekinumab: 90 mg/mL + histidine and/or a salt thereof (in terms of histidine): 6.4 mM + various tonicity adjusting agents) were obtained in the same manner as in Comparative Example 2 except that various tonicity adjusting agents were further mixed. The various tonicity adjusting agents used were such that Comparative Example 10: sucrose (76 mg/mL) and Comparative Example 11: mannitol (30 mg/mL (solubility limit concentration at the time of preparation of the formulation)) at the concentrations in the parentheses.

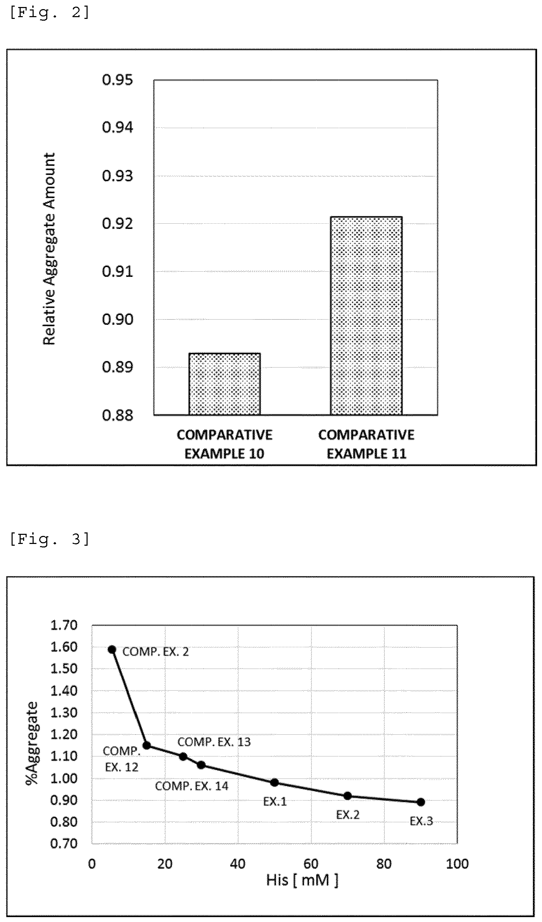

The storage stability evaluation test was carried out on each of the aqueous formulations obtained in Comparative Examples 10 and 11 to obtain the relative amount (relative aggregate amount) of the %aggregate of each aqueous formulation to the %aggregate of the aqueous formulation obtained in Comparative Example 2, in the same manner as in Example 1 and Comparative Examples 1 to 9. Fig. 2 illustrates a graph illustrating the relative aggregate amounts after storing the aqueous formulations at 40°C for 2 weeks. From the results illustrated in Fig. 2, it was confirmed that the generation of aggregates was suppressed in the case of adding sucrose to an ustekinumab-containing aqueous formulation.

### (Examples 2 and 3 and Comparative Examples 12 to 14)

The aqueous formulations were obtained in the same manner as in Example 1 except that the concentrations of histidine and/or a salt thereof were changed to obtain the compositions presented in Table 1 below. In addition, Examples 1 to 3 obtained aqueous formulations further added with L-methionine to adjust its concentration to 10, 25, and 50 mM. Table 1 below presents the compositions of the obtained aqueous formulations. In addition, Table 1 also presents the compositions of the aqueous formulations obtained in Example 1 and Comparative Example 2. Note that, in the tables below, Histidine [mM] indicates the content of histidine and/or a salt thereof in terms of histidine included due to the histidine buffer solution (hereinafter the same).

**[Table 1]**

| | Comparative Example 2 | Comparative Example 12 | Comparative Example 13 | Comparative Example 14 | Example 1 | Example 2 | Example 3 |
|---|---|---|---|---|---|---|---|
| Ustekinumab [mg/mL] | 90 | 90 | 90 | 90 | 90 | 90 | 90 |
| Histidine [mM] (Histidine Buffer Solution) | 6.4 | 15 | 25 | 30 | 50 | 70 | 90 |
| Methionine [mM] | - | - | - | - | 0, 10, 25, 50 | 0, 10, 25, 50 | 0, 10, 25, 50 |
| pH | 6.0 | 6.0 | 6.0 | 6.0 | 6.0 | 6.0 | 6.0 |

The storage stability evaluation test was carried out on each of the aqueous formulations obtained in Example(EX.)s 1 to 3 and Comparative Example(COMP.EX.)s 2 and 12 to 14. Fig. 3 illustrates a graph illustrating the %aggregate after each of the aqueous formulations (methionine concentration: 0 mM) was stored at 40°C for 2 weeks, that is, a graph illustrating the relationship between the %aggregate (%Aggregate) and the histidine concentration (His [mM]) after storage at 40°C for 2 weeks. As is apparent from the results illustrated in Fig. 3, it was confirmed that the generation of aggregates is significantly suppressed and excellent safety can also be expected in the aqueous formulations of the present invention (Examples 1 to 3) having a concentration of histidine and/or a salt thereof (His [mM]) in a specific range.

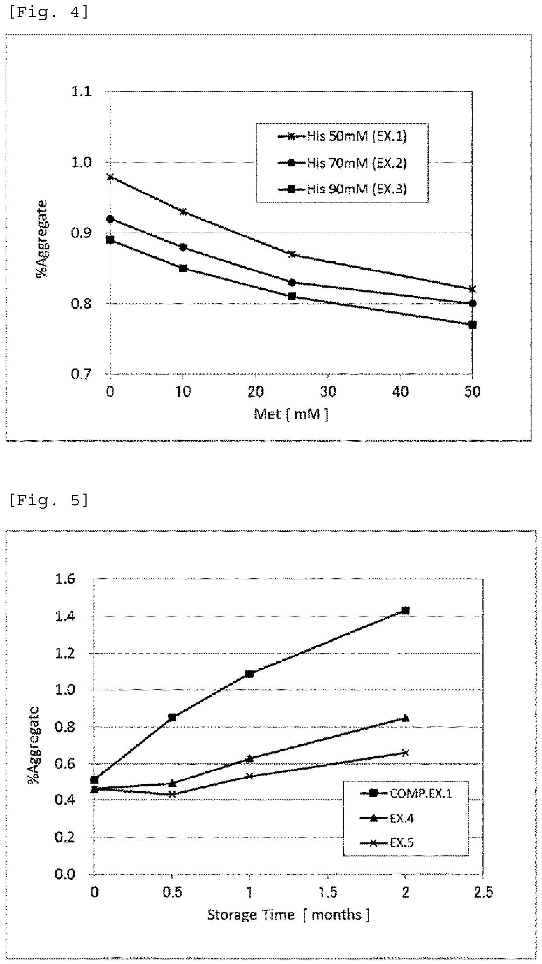

In addition, Fig. 4 illustrates a graph illustrating the relationship between the %aggregate (%Aggregate) and the methionine concentration (Met [mM]) after storage at 40°C for 2 weeks in the aqueous formulations obtained in Examples 1 to 3 (methionine concentration: 0, 10, 25, and 50 mM). As is apparent from the results illustrated in Fig. 4, it was confirmed that the generation of aggregates was further suppressed in the case of further adding methionine in the aqueous formulations of the present invention (Examples 1 to 3).

### (Examples 4 and 5)

First, a histidine buffer solution (pH 6.0) was prepared in the same manner as in Example 1. Subsequently, ustekinumab, L-methionine, the histidine buffer solution, Polysorbate 80, and sucrose were mixed to the compositions presented in Table 2 below, to thereby obtain 500 µL of aqueous formulation. Table 2 below presents the compositions of the obtained aqueous formulations. In addition, Table 2 also presents the composition of the aqueous formulation obtained in Comparative Example 1.

**[Table 2]**

| | Comparative Example 1 | Example 4 | Example 5 |
|---|---|---|---|
| Ustekinumab [mg/mL] | 90 | 90 | 90 |
| Histidine [mM] (Histidine Buffer Solution) | 6.4 | 70 | 70 |
| Methionine [mM] | - | - | 25 |
| Polysorbate 80 [mg/mL] | 0.04 | 0.04 | 0.04 |
| Sucrose [mg/mL] | 76 | 51.8 | 44.7 |
| pH | 6.0 | 6.0 | 6.0 |

The storage stability evaluation test was carried out on each of the aqueous formulations obtained in Examples 4 and 5 and Comparative Example 1. Fig. 5 illustrates a graph illustrating the relationship between the %aggregate (%Aggregate) and the storage time (Storage Time [weeks]) immediately after production of the aqueous formulations and after storage for 2 weeks, 1 month, and 2 months at 40°C. As is apparent from the results illustrated in Fig. 5, it was confirmed that the generation of aggregates was sufficiently suppressed even in the case of storage for a long period of time in the aqueous formulations of the present invention (Examples 4 and 5).

### (Examples 6 to 9 and Comparative Example 15)

First, a histidine buffer solution (pH 6.0) was prepared in the same manner as in Example 1. In addition, ustekinumab obtained in the same manner as in Example 1 was stored at 37°C for 24 hours for aggregation, and was formed into aggregated ustekinumab. Subsequently, the aggregated ustekinumab, L-methionine, the histidine buffer solution, Polysorbate 80, and sucrose were mixed to the compositions presented in Table 3 below, to thereby obtain 500 µL of aqueous formulation. Table 3 below presents the compositions of the obtained aqueous formulations.

**[Table 3]**

| | Comparative Example 15 | Example 6 | Example 7 | Example 8 | Example 9 |
|---|---|---|---|---|---|
| Aggregated Ustekinumab [mg/mL] | 90 | 90 | 90 | 90 | 90 |
| Histidine [mM] (Histidine Buffer Solution) | 6.4 | 60 | 70 | 60 | 70 |
| Methionine [mM] | - | - | - | 25 | 25 |
| Polysorbate 80 [mg/mL] | 0.04 | 0.04 | 0.04 | 0.04 | 0.04 |
| Sucrose [mg/mL] | 76 | 54.6 | 51.8 | 44.7 | 44.7 |
| pH | 6.0 | 6.0 | 6.0 | 6.0 | 6.0 |

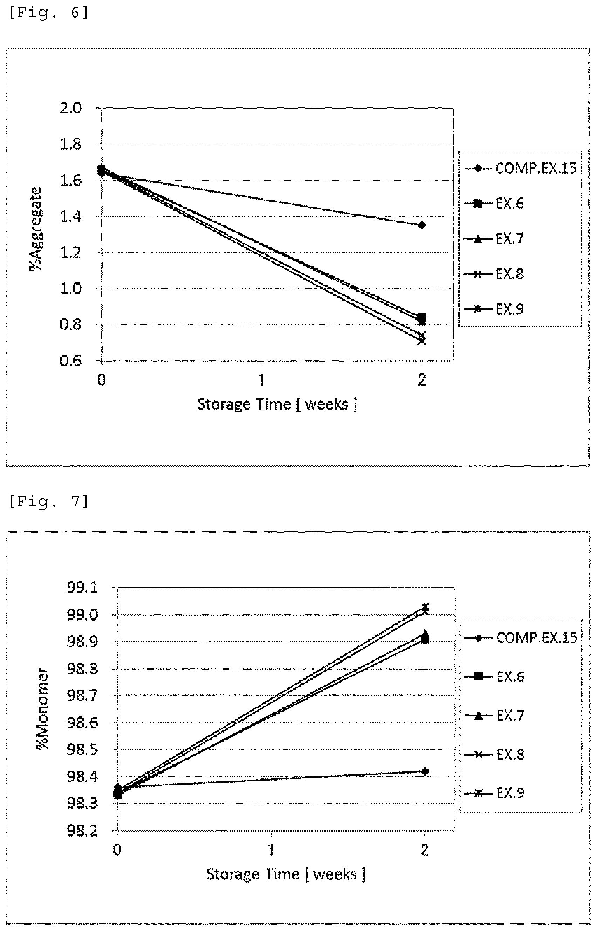

The storage stability evaluation test was carried out on each of the aqueous formulations obtained in Examples 6 to 9 and Comparative Example 15. Fig. 6 illustrates a graph illustrating the relationship between the %aggregate (%Aggregate) and the storage time (Storage Time [weeks]) immediately after production of the aqueous formulations and after storage for 2 weeks at 40°C. In addition, Fig. 7 illustrates a graph illustrating the relationship between the %monomer (%Monomer) and the storage time (Storage Time [weeks]) immediately after production of the aqueous formulations and after storage for 2 weeks at 40°C. As is apparent from the results illustrated in Figs. 6 and 7, it was confirmed that the aggregated ustekinumab was deaggregated in the aqueous formulations of the present invention (Examples 6 to 9).

### [Industrial Applicability]

The present invention makes it possible to provide an ustekinumab-containing aqueous formulation excellent in stability and an in-syringe aqueous formulation containing the same, and an antibody protein deaggregation agent capable of deaggregating aggregated ustekinumab and an antibody protein deaggregation method. For this reason, it is also expected to improve the stability of the ustekinumab-containing aqueous formulation, to reduce the suppression of the pharmacological action, and further to improve safety such as reduced immunogenicity.

## Claims

1. An aqueous formulation comprising:
ustekinumab;
at least one selected from histidine and salts thereof; and
water, wherein
a content of the ustekinumab is 80 to 100 mg/mL,
a content of the histidine and/or the salts thereof is 50 to 90 mM in terms of histidine, and
a pH thereof is 5.7 to 6.3.

2. The aqueous formulation according to claim 1, further comprising methionine, wherein a content of the methionine is 10 to 50 mM.

3. The aqueous formulation according to claim 1 or 2, further comprising sucrose.

4. The aqueous formulation according to any one of claims 1 to 3, formed by blending the ustekinumab and a histidine buffer solution.

5. An in-syringe aqueous formulation comprising a syringe and the aqueous formulation according to any one of claims 1 to 4 filled in the syringe.

6. An antibody protein deaggregation method for deaggregating aggregated ustekinumab, comprising the step of:
adding an antibody protein deaggregation agent to the aggregated ustekinumab to obtain an aqueous formulation according to claim 1,
wherein the antibody protein deaggregation agent deaggregates aggregated ustekinumab and comprises:
at least one selected from histidine and salts thereof; and water,
wherein a content of the histidine and/or the salts thereof is 50 to 90 mM in terms of histidine, and a pH thereof is 5.7 to 6.3.

7. The antibody protein deaggregation method of claim 6, wherein the antibody protein deaggregation agent further comprises methionine, wherein a content of the methionine is 5 to 50 mM.

8. The antibody protein deaggregation method of claim 6 or 7, wherein the antibody protein deaggregation agent further comprises sucrose.

## Patentansprüche

1. Wässrige Formulierung, umfassend:
Ustekinumab,
wenigstens eines, ausgewählt aus Histidin und Salzen davon; und
Wasser, wobei
ein Gehalt des Ustekinumabs 80 bis 100 mg/ml beträgt,
ein Gehalt des Histidins und/oder der Salze davon 50 bis 90 mM, bezogen auf Histidin, beträgt und
ein pH-Wert davon von 5,7 bis 6,3 beträgt.

2. Wässrige Formulierung nach Anspruch 1, ferner umfassend Methionin, wobei ein Gehalt des Methionins 10 bis 50 mM beträgt.

3. Wässrige Formulierung nach Anspruch 1 oder 2, ferner umfassend Saccharose.

4. Wässrige Formulierung nach einem der Ansprüche 1 bis 3, ausgebildet durch Vermischen des Ustekinumabs und einer Histidin-Pufferlösung.

5. Wässrige In-Spritze-Formulierung, umfassend eine Spritze und die wässrige Formulierung nach einem der Ansprüche 1 bis 4, die in die Spritze gefüllt ist.

6. Antikörperproteindeaggregationsverfahren zum Deaggregieren von aggregiertem Ustekinumab, umfassen die Schritte:
Hinzufügen eines Antkörperproteindeaggregationsmittels zu dem aggregierten Ustekinumab, um eine wässrige Formulierung nach Anspruch 1 zu erhalten,
wobei das Antkörperproteindeaggregationsmittel aggregiertes Ustekinumab deaggregiert und umfasst:
wenigstens eines, ausgewählt aus Histidin und Salzen davon; und Wasser,
wobei ein Gehalt des Histidins und/oder der Salze davon 50 bis 90 mM, bezogen auf Histidin, beträgt und ein pH-Wert davon von 5,7 bis 6,3 beträgt.

7. Antikörperproteindeaggregationsverfahren nach Anspruch 6, wobei das Antkörperproteindeaggregationsmittel ferner Methionin umfasst, wobei ein Gehalt des Methionins 5 bis 50 mM beträgt.

8. Antikörperproteindeaggregationsverfahren nach Anspruch 6 oder 7, wobei das Antkörperproteindeaggregationsmittel ferner Saccharose umfasst.

## Revendications

1. Préparation aqueuse, comprenant :
de l'ustékinumab ;
au moins un sélectionné parmi de l'histidine et des sels de celle-ci ; et
de l'eau, dans laquelle
une teneur en l'ustékinumab est de 80 à 100 mg/mL,
une teneur en l'histidine et/ou les sels de celle-ci est de 50 à 90 mM quant à l'histidine, et
un pH de celle-ci est de 5,7 à 6,3.

2. Préparation aqueuse selon la revendication 1, comprenant en outre de la méthionine, dans laquelle une teneur en la méthionine est de 10 à 50 mM.

3. Préparation aqueuse selon la revendication 1 ou 2, comprenant en outre du saccharose.

4. Préparation aqueuse selon l'une quelconque des revendications 1 à 3, formée en mélangeant l'ustékinumab et une solution tampon d'histidine.

5. Préparation aqueuse en seringue, comprenant une seringue et la préparation aqueuse selon l'une quelconque des revendications 1 à 4 remplissant la seringue.

6. Procédé de désagrégation de protéine anticorps pour désagrégation d'ustékinumab agrégé, comprenant l'étape de :
l'ajout d'un agent de désagrégation de protéine anticorps à l'ustékinumab agrégé pour obtenir une préparation aqueuse selon la revendication 1,
dans lequel l'agent de désagrégation de protéine anticorps désagrège l'ustékinumab agrégé et comprend :
au moins un sélectionné parmi de l'histidine et sels de celle-ci ; et de l'eau,
dans lequel une teneur en l'histidine et/ou les sels de celle-ci est de 50 à 90 mM quant à l'histidine, et un pH de celle-ci est de 5,7 à 6,3.

7. Procédé de désagrégation de protéine anticorps de la revendication 6, dans lequel l'agent de désagrégation de protéine anticorps comprend en outre de la méthionine, dans lequel une teneur en la méthionine est de 5 à 50 mM.

8. Procédé de désagrégation de protéine anticorps de la revendication 6 ou 7, dans lequel l'agent de désagrégation de protéine anticorps comprend en outre du saccharose.
